# EUROPEAN PATENT SPECIFICATION

(11) **EP 1 530 592 B1**
(45) Date of publication and mention of the grant of the patent: **17.12.2008**
(21) Application number: 03792325.7
(22) Date of filing: 14.08.2003
(51) Int. Cl.: C07K 14/74, C07K 14/78, C12N 15/62, C12N 15/12, A61K 38/17, G01N 33/68

(54) **CHIMERIC MHC PROTEIN AND OLIGOMER THEREOF**
CHIMÄRES MHC-PROTEIN UND OLIGOMER DAVON
PROTEINE CHIMERIQUE MHC ET SON OLIGOMERE

(30) Priority: 21.08.2002 GB 0219459
(43) Date of publication of application: 18.05.2005
(73) Proprietor: ProImmune Limited, Oxford OX4 4GA (GB)
(72) Inventor: SCHWABE, Nikolai, Franz, Gregor, Oxford OX2 6BP (GB); CHENG-CHOO TAN, Linda, Oxford OX2 6BP (GB); NAPPER, Catherine, Elizabeth, Coggs, Whitney OX28 3UD (GB); FRY, Jeremy, William, Nuneham Courtney OX44 9PQ (GB); PANG, Susan, Lincoln LN2 2ES (GB); SPOONER, Rachel, Kate, Oxford OX3 0RU (GB)
(74) Representative: Vossius & Partner
(86) International application number: PCT/EP2003/009056
(87) International publication number: WO 2004/018520

(56) References cited:
- WO-A-98/06749
- WO-A-99/42597
- WO-A-02/055992
- US-A1- 2002 082 411
- MÜLLER K M ET AL: "Protein fusions to coiled-coil domains." METHODS IN ENZYMOLOGY. UNITED STATES 2000, vol. 328, 2000, pages 261-282, XP008025980 ISSN: 0076-6879
- HOLLER N ET AL: "Development of improved soluble inhibitors of FasL and CD40L based on oligomerized receptors" JOURNAL OF IMMUNOLOGICAL METHODS, ELSEVIER SCIENCE PUBLISHERS B.V.,AMSTERDAM, NL, vol. 237, no. 1-2, April 2000 (2000-04), pages 159-173, XP004192503 ISSN: 0022-1759 cited in the application
- TERSKIKH ALEXEY V ET AL: "Peptabody: A new type of high avidity binding protein" PROCEEDINGS OF THE NATIONAL ACADEMY OF SCIENCES OF THE UNITED STATES, NATIONAL ACADEMY OF SCIENCE, WASHINGTON, DC, US, vol. 94, no. 5, 1997, pages 1663-1668, XP002147182 ISSN: 0027-8424

## Description

The present invention relates to a chimeric MHC protein, an expression cassette encoding the same, a vector, an oligomer of said chimeric protein, a method of labeling, detecting and separating mammalian T cells according to the specificity of their antigen receptor by use of the oligomer, and suitable primers for constructing said chimeric protein.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells.

It has been established that isolated or recombinant forms of MHC-peptide molecules are useful for detecting, separating and manipulating T cells according to the specific peptide antigens these T cells recognize. It has also been understood that the interaction between MHC molecules and TCRs across cell surfaces is multimeric in nature and that the affinity of a single MHC molecule for a given TCR is generally low in affinity.

As a consequence, there has been an effort to develop multimeric forms of isolated or recombinant MHC-peptide molecules to make such molecules more useful in the applications described above.

European Patent Application EP 812 331 discloses a multimeric binding complex for labeling, detecting and separating mammalian T cells according to their antigen receptor specificity, the complex having the formula (α-β-P)n, wherein (α-β-P) is an MHC peptide molecule, n is ≥ 2, α comprises an α chain of a MHC I or MHC II class molecule, β comprises a β chain of an MHC protein and P is a substantially homogeneous peptide antigen. The MHC peptide molecule is multimerised by biotinylating the C terminus of one of the α or β chain of the MHC molecule and coupling of MHC monomers to tetravalent streptavidin/avidin or by providing a chimeric protein of an MHC molecule which is modified at the C terminus of one of the α or β chain to comprise an epitope which is recognised by a corresponding antibody that serves as a multimerising entity. The document further teaches use of the MHC oligomers for detecting, labeling and separating specific T cells according to their TCR specificity.

European Patent Application EP 665 289 discloses specific peptides, MHC molecules binding these peptides, and oligomers obtained by crosslinking of the respective MHC molecules having the specific peptide bound to them. Oligomerisation is achieved by using chemical crosslinking agents or by providing MHC chimeric proteins comprising an epitope, which is recognised by an immunoglobulin such as IgG or IgM. The MHC molecules may comprise a label and may be used for labeling, detecting, and separating T cells according to their specific receptor binding, and may eventually be employed in therapy of humans.

WO 93/10220 discloses a chimeric MHC molecule, comprising the soluble part of an MHC molecule, which can be either class I or class II MHC fused to an immunoglobulin constant region. The MHC portion of the molecule comprises complementary α and/or β chains and a peptide is bound in the respective binding grooves of the MHC molecules. Due to the presence of the dimeric immunoglobulin scaffold these chimeric MHC-Ig molecules undergo self-assembly into a dimeric structure.

In other research the oligomerisation domain of cartilage oligomeric matrix protein (COMP) has been used as a tool for multimerising several proteins in the past. COMP has been described and characterised by Efimov and colleagues (see e.g. Proteins: Structure, Function, and Genetics 24:259-262 (1996)). COMP is a pentameric glycoprotein of the thrombospondin family. Self-assembly of the protein to form pentamers is achieved through the formation of a five-stranded helical bundle that involves 64 N-terminal amino acid residues of the protein. The amino acid sequence of the oligomerisation domain has been disclosed by Efimov et al., FEBS Letters 341:54-58 (1994).

WO 00/44908 discloses chimeric proteins that contain anti-angiogenic portions of TSP-1, TSP-2, endostatin, angiostatin, platelet factor 4 or prolactin linked to a portion of the N-terminal region of human cartilage oligomeric matrix protein (COMP) thus allowing for the formation of pentamers. The document is predominantly concerned with exploiting the anti-angiogenic effect mediated by the resulting chimeric proteins. According to this disclosure the chimeric protein should promote correct folding of the TSP-domains contained therein, so that they better mimic the natural proteins than peptides that are based on the TSR sequence.

US 6,218,513 discloses globins containing non-naturally occurring binding domains for creating oligomers of said globins. The COMP oligomerisation domain is one of the disclosed binding domains. The advantage seen from oligomerisation relates to increased half-life and hence better resistance against intravasal degradation as well as reduced extravasation of the oligomerised globin proteins, due to their increased size compared to monomeric globin proteins.

Holler et al., Journal of Immunological Methods 237:159-173 (2000), disclose the development of improved soluble inhibitors of FasL and CD40L based on oligomerised receptors comprising TNF-receptor family members fused to the constant region of IgG or the self-assembling domain of COMP. It is concluded there that increased affinity of oligomeric soluble chimeric receptors of the TNF-receptor family is not a general phenomenon. It is found that the affinity of such oligomeric chimeric receptors to their ligand depends on the specific receptor-ligand pair under consideration and this is shown to vary significantly even between closely related proteins.

WO 02/055992 discloses self-multimerizing MHC fusion proteins and oligomeric complexes thereof.

The attempts for multimerisation of MHC proteins described hereinbefore pose several disadvantages. Chemical crosslinking for example typically results in a non-predictable structure of the final MHC oligomer, which may vary considerably for each complex. Hence, binding to the target may vary likewise, depending on the final oligomer structure. This in turn can impede accuracy and reliability of any assay system the oligomers are used in. In the worst case chemical crosslinking may even prevent formation of a functional MHC oligomer altogether.

Fusing either one or two of the MHC polypeptide chains with the constant region of an immunoglobulin molecule such as described in WO 93/10220 results in a dimeric MHC molecular complex. Although the dimeric interaction can contribute to increasing the affinity of the complex, further multimerisation through anti-idiotypic antibodies or protein A or G may be required to reach the affinity required for various applications, such as detecting antigen specific T cells or activating such cells successfully. Such two-stage multimerisation processes can, however, be time-consuming to carry out and it is difficult to control the uniformity of the final product.

Multimerisation of the MHC monomers by use of non-human binding partners such as the biotin/streptavidin binding pair or non-human antibodies on the other hand introduces non-human protein components into the oligomer. This raises concerns with regard to potential toxicity of the complex and/or immune responses against this non-human part of the complex in applications where in vivo use is envisaged, e.g. in human therapy. Accordingly it would be desirable to avoid non-human portions in the oligomeric complex.

In addition producing MHC multimers that rely on the biotin-streptavidin interaction involves a substantial number of process steps, including several rounds of protein purification, and a biotinylation reaction that can lead to significant loss of active material. Further, controlling the biotinylation efficiency of monomeric MHC subunits and quality of the final multimeric product is difficult.

The MHC oligomers available from the art provide for a certain enhancement of affinity of the complex when compared to the MHC monomer itself. A further increase in affinity would, however, be very desirable without increasing the complexity of the synthesis of the complexes while assuring that such synthesis will yield molecules with high uniformity.

It is the object of the present invention to overcome the above drawbacks and other disadvantages of the prior art and to provide a multimeric MHC peptide complex that is easy to manufacture with a uniformly high valency of MHC peptide components that are available simultaneously for binding to T cell receptors and a protein sequence that minimizes non-human content.

### Summary of the Invention

The above disadvantages and drawbacks of the prior art are overcome by using at least two chimeric proteins comprising a first section derived from an MHC peptide chain or a functional part thereof and a second section comprising a pentamerisation domain derived from the pentamerisation domain of a cartilage oligomeric matrix protein (COMP) to form an oligomeric MHC complex.

A first aspect of the present invention therefore concerns an oligomeric MHC complex comprising at least two chimeric proteins, said chimeric proteins comprising a first section derived from an MHC peptide chain or a functional part thereof and a second section comprising a pentamerisation domain derived from COMP, wherein formation of the oligomeric MHC complex occurs by oligomerisation at the pentamerisation domain of the chimeric proteins, and wherein at least two of the first sections of all chimeric proteins comprised in said oligomer are derived from the same MHC peptide chain.

Preferably the first section of the chimeric proteins is derived from the extra-cellular part of the MHC class I or II α chain. Alternatively, the first section of the chimeric proteins may be derived from the extra-cellular part of the MHC class I or II β chain.

In a preferred embodiment of the invention the pentamerisation domain of COMP comprised in the second section of the chimeric proteins comprises and preferably consists of the amino acids 1 to 128, preferably 20 to 83, preferably 20 to 72 of said protein, most preferably 21-85 of human COMP.

Preferably, the chimeric proteins further comprise a first linker between the MHC peptide chain (first section) and the oligomerising domain (second section).

Preferably, at least one of the chimeric proteins in the oligomer further comprises one or more domains selected from the group consisting of a second linker, a tagging domain and a purification domain.

Another embodiment of the invention concerns an oligomeric MHC complex as described above that further comprises the complementary MHC peptide chain to at least two of the chimeric proteins to form functional MHC binding complexes.

Preferably the oligomeric MHC complex according to the first aspect of the invention comprises peptide bound to the MHC portions of the complex in the groove formed by the MHC α1 and α2 domains for class I complexes or the MHC α1 and β1 domains for class II complexes. Preferably the peptide is substantially homogeneous.

The oligomeric MHC complex according to the invention may comprise a label. Preferably, the label is selected from the group consisting of a light detectable label, a radioactive label, an enzyme, an epitope, a lectin, or biotin.

In a second aspect of the invention the same concerns a chimeric protein comprising a first section which is an MHC peptide chain or a functional part thereof and a second section comprising a pentamerisation domain derived from the pentamerisation domain of COMP. More preferably, the pentamerisation domain comprises and preferably consists of the amino acids 1 to 128, preferably 20 to 83 of human COMP, most preferably 20 to 72 of COMP.

A third aspect of the invention concerns a recombinant expression cassette comprising a promoter sequence operably linked to a nucleotide sequence coding for a chimeric protein for incorporation into the oligomeric MHC complex of the invention as described above.

A fourth aspect of the invention concerns a vector comprising the recombinant expression cassette described above.

A fifth aspect of the invention concerns a pharmaceutical or diagnostic composition, comprising an oligomeric MHC complex of the invention.

A sixth aspect of the invention concerns a method of labeling and or detecting mammalian T cells according to the specificity of their antigen receptor, the method comprising
(i) combining an oligomeric MHC complex according to the invention and a suspension or biological sample comprising T cells, and
(ii) detecting the presence of specific binding of said complex and the T cells.

A seventh aspect of the invention concerns a method of separating mammalian T cells according to the specificity of their antigen receptor, the method comprising
(i) combining an oligomeric MHC complex as defined above and a suspension or biological sample comprising T cells, and
(ii) separating T cells bound to said complex from unbound cells.

Also disclosed are primers useful in constructing the chimeric peptides described above.

### Description of the Drawings

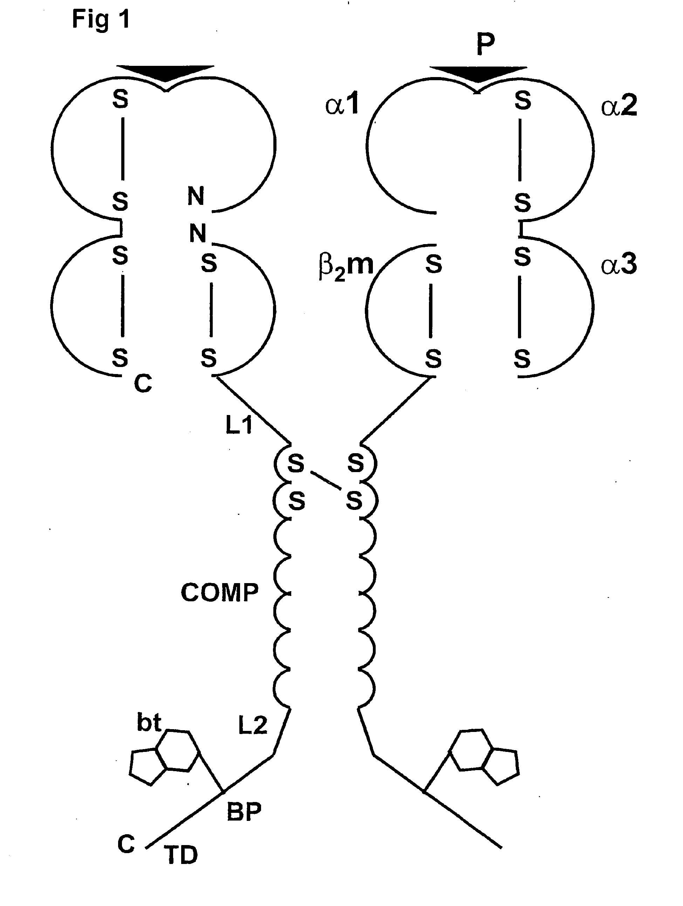

Fig.1 is a schematic drawing of an oligomeric MHC class I complex. The figure shows two out of several subunits of the fully assembled oligomeric class I chimeric complex. As shown β2-microgobulin (β2m) is fused to the coiled oligomerisation domain (OD) of an oligomer-forming coiled-coil protein and spaced from this domain by a first linker (L1). A further linker (L2) is provided at the C terminal end of the domain, followed by a recognition sequence (BP) for biotinylation by biotin-protein ligase BirA and finally a tagging/purification domain (TD) for purification and detection. A biotin molecule (bt), attached to the Lysine residue of the recognition sequence in form of a biotinylation peptide (BP) is also shown. The complementary MHC class I α chain having the domains α1, α2, and α3, is assembled with β2m and antigenic peptide (P). Disulphide bonds (S-S), stabilizing the MHC portion of the complex are indicated. Further disulphide bonds are also shown, illustrating the specific case where the oligomerising domain is derived from COMP, which will assemble into a stable pentamer, wherein these disulphide bonds link each pair of the COMP domains in the pentamer. The amino and carboxyl termini of the respective α and β in the complex are indicated by N and C, respectively.

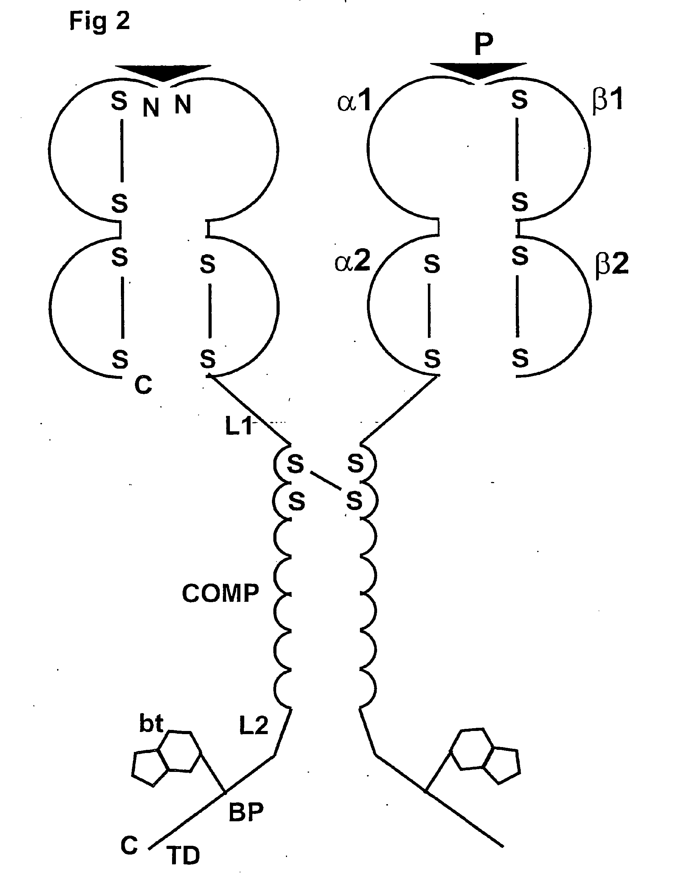

Fig. 2 is a schematic drawing of an oligomeric MHC class II complex. The figure generally follows the nomenclature of Fig. 1 with the difference that (β2m) is replaced by the MHC class II α chain, having the domains α1, α2, fused to the oligomerising domain via the linker (L1). Here the α chain is assembled with its complementary MHC class II β chain, having the domains β1, β2.

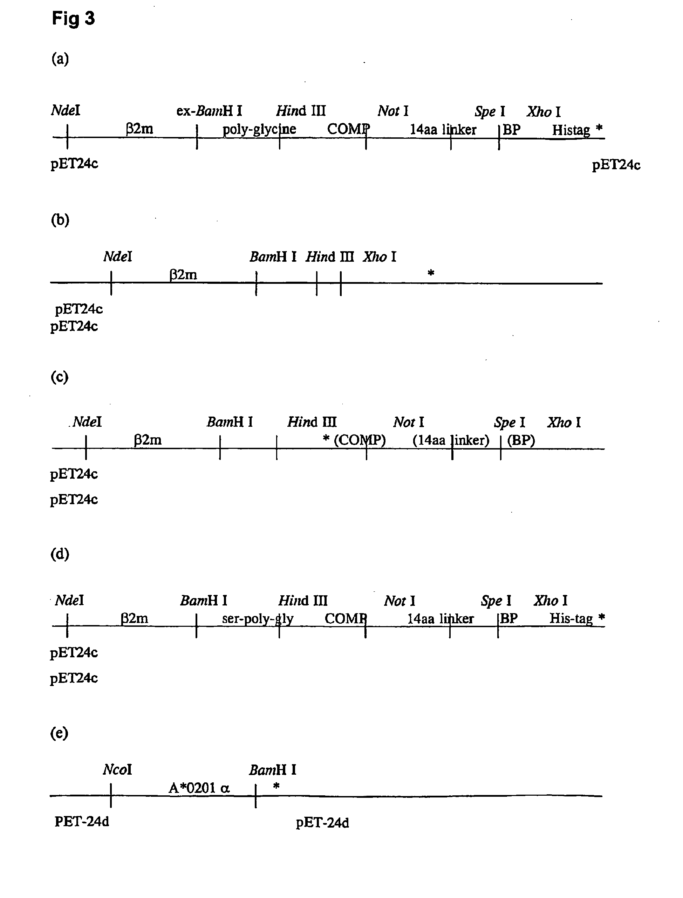

Fig. 3 shows a set of schematic maps, including restriction sites, illustrating (a) pETBMC04: the β2m-COMP expression construct, as well as (b)-(d) pETBMC01-03: β2m-COMP intermediate molecular cloning constructs, and finally (e) pETA2sol: the HLA-A*0201 α chain expression construct, as described in detail later on. Stop codons are indicated by the '*' symbol for each construct in (a)-(e).

### Detailed Description of the Invention

The oligomeric MHC complex of the invention allows for overcoming the disadvantages and drawbacks of the prior art. More specifically, the inventors have found that the above oligomeric MHC complex can have a substantially higher affinity to a T cell receptor than an oligomer obtained by e.g. tetramerising the MHC complexes by coupling through biotin and streptavidin. Without wishing to be bound by theory it is believed that this increase in affinity is achieved when three or more MHC molecules are arranged substantially in the same plane with all binding faces oriented in the same direction. In contrast, because the tetrameric streptavidin-coupled complex has a tetrahedral arrangement, at best only three MHC binding domains are available simultaneously for contacting the T cell surface. The complex according to the invention further allows for effective labeling while minimizing the interference of the chosen label with the active MHC domains, as the label(s) are located on the opposite end of the oligomer-forming coiled-coil domain.

The meaning of an "oligomer-forming coiled-coil protein" is a protein comprising an oligomerising domain. Said domains comprise two or more polypeptide subunits that may or may not be identical. The subunit of two or more of such oligomerisation domains assemble with one another such that each subunit in the oligomerising domain assumes a substantially helical conformation in the assembled state, wherein the subunits in the oligomerisation domain are arranged along an identifiable axis, wherein the oligomerising domain has two identifiable opposite ends along said axis, and wherein at least two of the polypeptide subunits have the same amino-to-carboxyl orientation along said axis. Corresponding oligomerisation domains and oligomer-forming coiled-coil proteins are known in the art as e.g. cited in the introductory portion hereof.

The oligomerising domain may be derived from a suitable oligomer-forming coiled-coil protein of any species. Preferably, the MHC molecule is fused to an oligomerisation domain of a human version oligomer-forming coiled-coil protein, in which case unwanted immune responses and/or rejection reactions are minimised in situations where the complex is to be administered to humans.

Examples for oligomer-forming coiled-coil proteins include various types of collagen, triple coiled-coil domains of C-type lectins, such as mannose binding protein (MBP); CIq, myosin, leucine zippers such those occurring in p53, GCN4, bacteriophage P22 Mnt repressor; and the trombospondin family proteins such as COMP. Preferably the oligomerisation domain is derived from the cartilage oligomeric matrix protein COMP. More preferably, the MHC molecule is fused to an oligomerisation domain of the human version of COMP for the reasons described above.

The number of chimeric proteins (n) comprised on the oligomeric MHC complex will typically depend on the type of oligomerisation domain the second section of the chimeric proteins is derived from and can in general be 2 or more, preferably n = 2 to 10, most preferably n = 3 or 5. If the second section of the chimeric proteins to be oligomerised is e.g. derived from the pentamerisation domain of the COMP this number will typically be five such that the oligomer will be a pentamer (n = 5), whereas in case these oligomerisation domains are derived from collagen this number will be three (n = 3).

Disclosed herein is an oligomeric MHC complex comprising chimeric proteins comprising a first section derived from an MHC peptide chain or a functional part thereof and a second section comprising an oligomerisation domain of an oligomer-forming coiled-coil protein. Although the MHC peptide chain of the first section preferably forms the N terminal part of the chimeric protein, whereas the oligomerising domain of the second section is located in the C terminal region thereof, both sections can in general be in any order. The preferred arrangement will, however, allow the MHC part to maintain its functional characteristics for all cases.

The term "chimeric protein" as used herein means a single peptide protein, the amino acid sequence of which is derived at least in part from two different naturally occurring proteins or protein chain sections, in this case an MHC peptide and a peptide substantially comprising at least a significant proportion of an oligomerising domain. With the term "a functional part thereof" as used herein, a part of a peptide chain is meant, which still exhibits the desired functional characteristics of the full-length peptide it is derived from. For MHC therefore a peptide chain is meant, which, if necessary, when completed or assembled with the complementary MHC peptide chain thereof, is capable of binding an antigenic peptide. With the term "complementary" MHC peptide chain the respective other peptide chain of a naturally occurring MHC complex is meant. The complementary chain to an a chain of the MHC complex is the β chain and *vice versa.*

The MHC peptide chain in the chimeric proteins may be the extra-cellular part of the MHC class I or II α chain. Alternatively, the MHC peptide chain may be the extra-cellular part of an MHC class I or II β chain. The MHC proteins may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 -microglobulin. Included in the murine H-2 subunits are the class I H-2K, H-2D, H-2L, and the class II I-Aα, I-Aβ, I-Eα and I-Eβ, and β2-microglobulin. Amino acid sequences of some representative MHC proteins are referenced in EP 812 331.

The MHC peptide chains may correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain. For class II proteins the soluble form will include the α1 and α2 or β1 and β2 domains of the α chain or β chain, respectively.

Not more than about 10, usually not more than about 5, preferably none of the amino acids of the transmembrane domain will be included. The deletion may extend as much as about 10 amino acids into the α3 domain. Preferably none of the amino acids of the α3 domain will be deleted. The deletion will be such that it does not interfere with the ability of the α3 domain to fold into a functional disulfide bonded structure. The class I β chain, β2m, lacks a transmembrane domain in its native form, and does not have to be truncated. Generally, no class II subunits will be used in conjunction with class I subunits.

The above deletion is likewise applicable to class II subunits. It may extend as much as about 10 amino acids into the α2 or β2 domain, preferably none of the amino acids of the α2 or β2 domain will be deleted. The deletion will be such that it does not interfere with the ability of the α2 or β2 domain to fold into a functional disulfide bonded structure.

One may wish to introduce a small number of amino acids at the polypeptide termini, usually not more than 25, more usually not more than 20. The deletion or insertion of amino acids will usually be as a result of the requirements in cloning, e.g. as a consequence of providing for convenient restriction sites or the like, and to manage potential steric problems in the assembly of the molecules. In addition, one may wish to substitute one or more amino acids with a different amino acid for similar reasons, usually not substituting more than about five amino acids in any one domain.

According to the present invention the MHC peptide chain comprised in the first section of a chimeric protein as defined above is fused, preferably at its C terminal end, to the pentamerising domain of COMP, which comprises, more preferably consists of the N terminal amino acids 1 to 128, preferably 20 to 83, most preferably 20 to 72 of said protein as discussed in the prior art section of this invention disclosure. With regard to numbering of amino acids, reference is made to Efimov et al., FEBS Letters 341:54-58 (1994). Further, similar to the MHC part of the chimeric protein of the invention, this domain can be altered by amino acid substitution, deletion or insertion, as long as the self-assembly of the oligomerising domain is not impaired.

Fusion of both peptide chains, MHC and the oligomerising domain, respectively, may be direct or, as is shown in Figures 1 and 2, may include a first linker (L1) connecting and spacing apart the MHC peptide and the oligomerising domain (OD) in the chimeric protein. Joining by use of a linker is preferred. In general such linker will comprise not more than 30, preferably not more than 26 amino acids. Suitable linkers are known in the art and include e.g. immunoglobulin hinge regions, or serine glycine repeat sequences.

As is further shown in Figures 1 and 2, the chimeric protein of the invention may further include, preferably at its C terminal end, one or more of a second linker (L2), a biotinylation peptide (BP) and a tagging domain and/or a purification domain (TD) in either order. In a preferred embodiment the chimeric protein of the invention includes all three of the above domains in this order. In general the second linker will comprise not more than 25, preferably not more than 20 amino acids and can be the same as detailed for the first linker above.

The tagging domain optionally included in the chimeric protein of the invention can be any domain which allows for labeling of the protein. Preferably the tagging domain includes a label. This label can be included in the domain itself such as an epitope recognised by an antibody or a light detectable or radioactive label. Preferably, the label is selected from the group consisting of fluorescent markers, such as such as FITC, phycobiliproteins, such as R- or B-phycoerythrin, allophycocyanin, Cy3, Cy5, Cy7, a luminescent marker, a radioactive label such as ¹²⁵I or ³²P, an enzyme such as horseradish peroxidase, or alkaline phosphatase e.g. alkaline shrimp phosphatase, an epitope, a lectin or biotin/streptavidin.

Where the label is itself a protein, the polypeptide chain of the protein used for labeling can be fused to the chimeric protein, preferably at its C terminus, to the label protein. For example a fluorescent protein such as a green fluorescent protein (GFP), or a subunit of a phycobiliprotein could be used in this chimeric. GFP chimeric protein technology is well known in the art. Chimeric proteins comprising a suitable domain from a phycobiliprotein is described, for example in WO 01/46395.

Alternatively the label may be attached at a specific attachment site provided in the tagging domain. For example, the tagging domain may include a recognition site for the biotin protein ligase BirA to allow for site-specific biotinylation of the tagging domain and hence recognition by streptavidin/avidin. Suitable recognition sequences for BirA are described in EP 711 303. Similarly a lectin may be attached, or any other site-specific enzymatic modification may be made by means of incorporating an amino acid recognition sequence for a modifying enzyme into the amino acid sequence of the chimeric proteins of the invention. Other possible types of enzymatic modification are described in EP812 331. Alternatively labeling can be achieved by binding of a suitably labeled antibody, or antibody fragment, such as a labeled F(ab) fragment.

The purification domain optionally to be included in the chimeric protein of the invention can be any domain assisting in purification of the protein of the invention e.g. by providing specific binding characteristics. Appropriate sequences are known to the skilled worker and can be applied as long as they do not interfere with the functional MHC and oligomerising domains of the chimeric protein. Preferably the purification domain is a hexahistidine sequence.

Disclosed herein is an oligomeric MHC complex formed by oligomerising through self-assembly of the oligomerising domain of an oligomer-forming coiled-coil protein in the second section of the above chimeric proteins. Oligomerisation of the oligomerising domain typically occurs spontaneously and results in stable oligomers of the chimeric proteins. Typically, the oligomeric complex will consist of several identical monomeric subunits (at least in their MHC part, depending on the oligomerisation domain). If desired, however, two or more chimeric proteins differing in their MHC portion may be admixed before oligomerisation. Thereby heterogeneous oligomers may be obtained. Generally, however, at least two of the chimeric proteins in each complex will comprise a first section derived from the same MHC α or β subunit. For n > 2 the oligomer may contain at least two of the chimeric proteins in each complex which comprise a first section derived from the same MHC molecule.

Preferably the oligomerising domain of COMP is used and results in spontaneous assembly of stable pentamers of the chimeric protein.

The oligomeric MHC complex may further comprise the complementary MHC peptide chain(s) to form functional MHC binding complexes as discussed above and may also comprise a peptide bound in the groove formed by the MHC α1 and α2 domains for class I MHC complexes or the MHC α1 and β1 domains for class II MHC complexes. Preferably the peptide is substantially homogeneous.

The antigenic peptides will be from about 6 to 14 amino acids in length for complexes with class I MHC proteins, and usually about 8 to 11 amino acids. The peptides will be from about 6 to 35 amino acids in length for complexes with class II MHC proteins, usually from about 10 to 20 amino acids. The peptides may have a sequence derived from a wide variety of proteins. In many cases it will be desirable to use peptides, which act as T cell epitopes. The epitope sequences from a number of antigens are known in the art.

Alternatively, the epitope sequence may be empirically determined by isolating and sequencing peptides bound to native MHC proteins, by synthesis of a series of putative antigenic peptides from the target sequence, then assaying for T cell reactivity to the different peptides, or by producing a series of MHC-peptide complexes with these peptides and quantification the T cell binding. Preparation of peptides, including synthetic peptide synthesis, identifying sequences, and identifying relevant minimal antigenic sequences is known in the art. In any case, the peptide comprised in the oligomeric MHC complex is preferably substantially homogeneous, meaning that preferably at least 80 % of the peptides are identical, more preferably at least 90 % and most preferably at least 95 %.

Also disclosed herein is a chimeric protein itself, which can be oligomerised into the oligomeric MHC complex of the invention. The chimeric protein comprises a first section derived from an MHC peptide chain or a functional part thereof and a second section comprising an oligomerising domain derived from an oligomer-forming coiled-coil protein which coiled-coil protein oligomerises by alignment of at least two substantially identical versions of the polypeptide chain from which the oligomerising domain is derived. In other words, the oligomer-forming coiled-coil protein used to derive the oligomerising domain in the chimeric protein of the present invention may be a homo-oligomer or a hetero-oligomer. Typically, however, it will comprise two or more copies of substantially identical polypeptide chain sections in its oligomerising domain that are aligned when the oligomer is formed. Preferably oligomerising domain derived from the pentamerisation domain of COMP. Preferred embodiments are as discussed above with regard to the oligomeric MHC complex itself.

In a third aspect thereof the present invention relates to a recombinant expression cassette comprising a promoter sequence operably linked to a first nucleotide sequence coding for an MHC peptide chain or a functional part thereof and a second nucleotide sequence encoding the oligomerising domain of COMP. In a first embodiment the first nucleotide sequence encodes the MHC peptide chain of the extra-cellular part of the MHC class I or II a chain. In a second embodiment the first nucleic acid sequence encodes the MHC peptide chain of the extra-cellular part of the MHC class I or II β chain. Preferably the second nucleotide sequence encodes amino acids 1 to 128, preferably 20 to 83, most preferably 20 to 72 of COMP. Preferably the COMP sequence is derived from human COMP.

Generally, the nomenclature used herein and the laboratory procedures in recombinant DNA technology described are those well known and commonly employed in the art. Standard techniques are used for DNA and RNA isolation, amplification, and cloning. Generally enzymatic reactions involving DNA ligase, DNA polymerase, restriction endonucleases and the like are performed according to the manufacturer's specifications, using enzyme buffers supplied by the manufacturer. These techniques and various other techniques are generally performed as known in the art. Suitable nucleotide sequences for the MHC and the oligomer-forming coiled coil domains such as the COMP domain are known in the art as discussed hereinbefore.

The DNA constructs will typically include an expression control DNA sequence, including naturally-associated or heterologous promoter regions, operably linked to protein coding sequences. The expression cassette may further include appropriate start and stop codons, leader sequences coding sequences and so on, depending on the chosen host. The expression cassette can be incorporated into a vector suitable to transform the chosen host and/or to maintain stable expression in said host.

The term "operably linked" as used herein refers to linkage of a promoter upstream from one or more DNA sequences such that the promoter mediates transcription of the DNA sequences. Preferably, the expression control sequences will be those eukaryotic or non-eukaryotic promoter systems in vectors capable of transforming or transfecting desired eukaryotic or non-eukaryotic host cells. Once the vector has been incorporated into the appropriate host, the host is maintained under conditions suitable for high-level expression of the nucleotide sequences, and the collection and purification of the chimeric proteins.

MHC-DNA sequences and DNA sequences for suitable oligomerising domains from an oligomer-forming coiled-coil protein, including that of COMP, can be isolated in accordance with well-known procedures from a variety of human or other cells. Traditionally, desired sequences are amplified from a suitable cDNA library, which is prepared from messenger RNA that is isolated from appropriate cell lines. Suitable source cells for the DNA sequences and host cells for expression and secretion can be obtained from a number of sources, such as the American Type Culture Collection (ATCC) or other commercial suppliers.

The nucleotide sequences or expression cassettes used to transfect the host cells can be modified according to standard techniques to yield chimeric molecules with a variety of desired properties. The molecules of the present invention can be readily designed and manufactured utilizing various recombinant DNA techniques well known to those skilled in the art. For example, the chains can vary from the naturally occurring sequence at the primary structure level by amino acid insertions, substitutions, deletions, and the like. These modifications can be used in a number of combinations to produce the final modified protein chain. In general, modifications of the genes encoding the chimeric molecule may be readily accomplished by a variety of well-known techniques, such as site-directed mutagenesis.

The amino acid sequence variants as discussed above can be prepared with various objectives in mind, including increasing the affinity of the molecule for target T cells, increasing or decreasing the affinity of the molecule for the respective CD4 or CD8 co-receptors interacting with class I or class II MHC complexes, for facilitating purification and preparation of the chimeric molecule or for increasing the stability of the complex, in vivo and *ex vivo.* The variants will, however, typically exhibit the same or similar biological activity as naturally occurring MHC molecules and retain and retain their oligomerising property of the corresponding naturally occurring oligomerising domain of the relevant oligomer-forming coiled-coil protein, respectively.

In addition, preparation of the oligomer is a straightforward process. For therapeutic uses this may be carried out in,mammalian cell culture, e.g. in CHO, COS, or human cell lines. Alternatively other expression systems can be used for expressing the molecules of the present invention, such as insect cell culture, including baculovirus and *Drosophila melanogaster* expression systems, yeast expression systems, or prokaryotic expression systems such as *Escherichia coli (E. coli).* If expression is performed in a prokaryotic expression system, either soluble expression, i.e. directed into the cytoplasm or periplasm, or insoluble expression, i.e. into inclusion bodies is possible.

A typical vector for *E. coli* would be one of the pET family of vectors which combine efficient expression from bacteriophage T7 RNA polymerase within an inducible *lac* operon-based system. The vectors containing the nucleotide segments of interest can be transferred into the host cell by well-known methods, depending on the type of cellular host. For example, transformation into chemical- or electro-competent cells is commonly utilised for prokaryotic cells, whereas calcium phosphate treatment, cationic liposomes, or electroporation may be used for other cellular hosts. Other methods used to transfect mammalian cells include the use of Polybrene, protoplast chimeric, microprojectiles and microinjection.

The chimeric protein and the complementary MHC α or β subunits, respectively, may be co-expressed and assembled as oligomeric complexes in the same cell. Alternatively these two components may be produced separately and allowed to associate *in vitro* in the presence of the antigenic peptide of choice to form stable oligomeric MHC peptide complexes. In the latter case mixing and association of the two components may occur either before or after oligomerisation of the chimeric peptide

The advantage of association of separate components *in vitro* is that oligomeric MHC-peptide complexes can be obtained with very high peptide homogeneity, e.g. greater than 95% or even greater than 99%. Where the complexes are expressed as fully assembled molecules, the peptide of interest can be introduced into the complexes, either by culturing the expressing cells with medium containing the antigenic peptide of interest, or by exchanging the peptide of interest with peptides that have endogenously bound during expression *in vitro,* which is typically done by incubating the purified complex with excess peptide at low or high pH so as to open the antigen binding pocket (see e.g. WO 93/10220). The antigenic peptide can also be covalently bound using standard procedures such as photoaffinity labeling (see e.g. WO 93/10220).

Alternatively, the peptide may be directly linked or fused to the expression construct of the chimeric protein or its complementary α or β chain. A suitable linker between the peptide portion and the N terminus of the chimeric protein or its complementary α or β chain, such as a polyglycine repeat sequence may be provided. Including the peptide as a chimeric peptide in the expression construct will allow for expression and folding of the complete MHC-peptide oligomeric complex without further addition of antigenic peptide or peptide exchange.

Conditions that permit folding and association of the subunits and chimeric proteins *in vitro* are known in the art. Assembly of class I MHC peptide complexes as well as assembly of functional class II complexes is e.g. described in EP 812 331. As one example of permissive conditions, roughly equimolar amounts of solubilised α and β subunits are mixed in a solution of urea in the presence of an excess of antigenic peptide of interest. In the case of class II MHC molecules refolding is initiated by dilution or dialysis into a buffered solution without urea. Peptides are loaded into empty class II heterodimers at about pH 5 to 5.5 over about 1 to 3 days, followed by neutralization, concentration and buffer exchange. Oligomerisation of the complex should occur simultaneously with formation of the α-β peptide complex. Alternatively a pentameric α or β complex may be achieved in two stages: first allowing oligomerisation of the oligomerising domain, followed by further refolding with the complementary α or β subunit in the presence of peptide according to the methods described above.

The assembled complex of the invention, or, initially the separate chimeric protein of the present invention and the complementary α or β subunit, can be purified according to standard procedures of the art, including ammonium sulfate precipitation, gel electrophoresis, column chromatography, including gel filtration chromatography, ion exchange chromatography, hydrophobic interaction chromatography, affinity chromatography, and the like.

The resulting peptide chain or oligomeric MHC complex of the present invention may be used therapeutically or in developing and performing assay procedures, immuno-stainings, and the like. Therapeutic uses of the oligomeric MHC complexes of the present invention require identification of the MHC haplotypes and antigens useful in treating a particular disease. In addition it may be necessary to determine the tissue types of patients before therapeutic use of the complexes according to the present invention, which is, however, a standard procedure well known in the art. The present invention is particularly suitable for treatment of cancers, infectious diseases, autoimmune diseases, and prevention of transplant rejection. Based on knowledge of the pathogenesis of such disease and the results of studies in relevant animal models, one skilled in the art can identify and isolate the MHC haplotype and antigens associated with a variety of such diseases.

In a fifth aspect the present invention therefore relates to a pharmaceutical or diagnostic composition, comprising the above oligomeric MHC complexes as defined above. Pharmaceutical compositions comprising the proteins are useful for, e.g. parenteral administration, i.e. subcutaneously, intramuscularly or intravenously. In addition, a number of new drug delivery approaches are being developed. The pharmaceutical compositions of the present invention are suitable for administration using these new methods, as well.

The compositions for parenteral administration will commonly comprise a solution of the chimeric protein or pentamer thereof dissolved in an acceptable carrier, preferably an aqueous carrier. A variety of aqueous carriers can be used, e.g. buffered water, 0.4 % saline, 0.3 % glycine and the like. These solutions are sterile and generally free of particulate matter. These compositions may be sterilised by conventional, well-known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of the chimeric protein in these formulations can vary widely, i.e. from less than about 1 pg/ml, usually at least about 0.1 mg/ml to as much as 10 - 100 mg/ml and will be selected primarily based on fluid volumes, viscosities, etc. in accordance with the particular mode of administration selected.

A typical pharmaceutical composition for intramuscular injection could be made up to contain 1 ml sterile buffered water, and 0.1 mg of oligomer complex protein. A typical composition for intravenous infusion could be made up to contain 250 ml of sterile Ringer's solution, and 10 mg of oligomer complex protein. Actual methods for preparing parenterally administrable compositions will be known or apparent to those skilled in the art.

The chimeric proteins or oligomeric MHC complexes of this invention can be lyophilised for storage and reconstituted in a suitable carrier prior to use. This technique has been shown to be effective and commonly used lyophilization and reconstitution techniques can be employed. It will be appreciated by those skilled in the art that lyophilization and reconstitution can lead to varying degrees of activity loss and that use levels may have to be adjusted to compensate.

Detecting or separating the T cells according to the specificity of their antigen receptor, as described hereinbefore, may involve any known technique. Suitable techniques are e.g. disclosed in EP 812 331. More in detail, the oligomeric MHC complexes of the invention can be used in labeling and detection of antigen specific T cells in suspension or other biological samples, such as in tissue samples, or may be used for separation of T cells e.g. when bound or immobilised on substrates, including paramagnetic or other beads as known to a skilled worker, or by flow cytometry.

Hence the complexes of the present invention can be used for example to purify and enrich T cells that are specific for a particular antigen and re-introducing these T cells to a patient autologously after expansion and/or other manipulation in cell culture in various disease situations. Alternatively T cells could be selectively depleted, e.g. in the case of an autoimmune disorder or other unwanted T cell immune responses.

Due to their enhanced affinity for specific T cell receptors, the oligomeric MHC complexes according to the present invention can allow for improved drug potency, better serum half-life and also improved pharmacokinetics, the latter presumably being due to increased molecular mass.

Due to the possibility of expressing the chimeric proteins and complementary MHC α or β portions comprised in the oligomeric MHC complexes of the invention in non-eukaryotic systems they are further easy to make at high yields, and can be expressed as sub-components, which can subsequently be refolded with one another in the presence of a homogeneous population of antigenic peptide.

These and other advantages are available to the skilled worker from the foregoing description. The following examples are given for the purpose of illustration only and shall not be construed as limiting the present invention in any way.

### Examples

The following is a detailed example for cloning, expressing, and purifying a pentameric class I MHC complex, which comprises a chimeric fusion of β2m with COMP. The chimeric β2m-COMP protein is expressed in insoluble inclusion bodies in *E. coli* and subsequently assembled as pentameric β2m-COMP *in vitro.* The pentameric class I MHC peptide complex is then formed in a second refolding reaction by combining β2m-COMP pentamers and the human MHC class I α molecule known as HLA-A*0201, in the presence of an appropriate synthetic binding peptide representing the T cell antigen. In this example, a well characterized antigen derived from Epstein-Barr virus BMLF1 protein, GLCTLVAML (a.a. 289-297), is used. The resultant complex is labelled with a fluorescent entity and used as a staining reagent for detecting antigen-specific T cells from a mixed lymphocyte population, in a flow cytometry application.

### 1. Molecular cloning of the β2m-COMP construct

The strategy involves the sequential cloning into pET-24c vector of β2m, yielding a construct referred to as pETBMC01, followed by the insertion of the oligomerisation domain of cartilage oligomeric matrix protein (COMP) with a biotin acceptor sequence (BP) for site-specific biotinylation with the biotin-protein ligase BirA, yielding a construct referred to as pETBMC02. Thirdly a polyglycine linker is cloned in between β2m and COMP, yielding a construct referred to as pETBMC03, and finally, a serine-residue is removed by site-directed mutagenesis, which serine residue precedes the poly-glycine linker, to give the final β2m-COMP/pET-24c construct, referred to as pETBMC04 (see also Figure 3). Removal of the serine residue is carried out to avoid steric hindrance when the β2m molecule is associated with the MHC class I αchain protein.

### 1.1. Cloning β2m into the pET-24c vector

### Source of β2m

The extracellular portion of β2m comprises of 99 amino acids (equivalent to Ile1-Met99 of the mature protein) encoded by 74 bp-370 bp of the DNA sequence. This region of the β2m sequence is amplified from a normal human lymphocyte cDNA library, by polymerase chain reaction (PCR) using the primers BMC#1 (1 µM) and BMC#2 (1 µM) which incorporate *Nde*I and *BamH*I restriction sites, respectively. (Sigma-Genosys, see Appendix I for primer sequences). Amplification is carried out using a proof-reading DNA polymerase (*Pfx*, Invitrogen) (1.25 U) in *Pfx* amplification buffer (as supplied by the manufacturer), supplemented with 2 mM MgSO₄ and 0.2 mM dNTPs following standard thermal cycling conditions. Step 1: 94°C, 4 min; Step 2: 94°C, 1 min; Step 3: 55°C, 1 min; Step 4: 72°C, 30 sec; Step 5: cycle to Step 2, 34x, Step 6: 72°C 10 min.

### Restriction enzyme digestion of β2m and pET-24c

β2m PCR product is purified from the above reaction mix using a QIAquick PCR purification kit according to the manufacturer's instructions (Qiagen). 200 ng of purified PCR product and 1 µg pET-24c vector (Novagen) are each digested with *Bam*H I (10 U) and *Nde* I (10 U) restriction enzymes (New England Biolabs, NEB) for 4 h at 37°C, in accordance with the manufacturer's instructions. The digested fragments are separated by horizontal gel electrophoresis in 1 % agarose (Bioline), and purified using a QIAEX II gel extraction kit, according to the manufacturer's instructions (Qiagen).

### Ligation of β2m into pET-24c (pETBMC01)

The gel-purified insert and vector DNA are ligated at a 1:3 molar ratio (vector:insert, 50 ng: 7.5 ng) using T4 DNA ligase (5 U; Bioline), in T4 DNA ligase buffer (as supplied) for 16 hrs at 16°C.

### Transformation of pETBMC01 into E. coli host strain XL1-Blue

The ligation mixtures and appropriate controls are subsequently transformed into XLI-Blue strain competent *E. coli* cells, according to the manufacturer's instructions (Stratagene). Successful transformants are selected by plating the cells on Luria-Bertani (LB) agar plates containing 30 µg/ml kanamycin, and incubating overnight at 37°C.

### PCR screening of transformants

A selection of single colonies from the bacterial transformation plates are screened by PCR with T7 promoter (1 µM) and T7 terminator (1 µM) primers (Sigma Genosys, see Appendix I for primer sequences), which are complementary to regions of the pET vector flanking the cloning site. Amplification is carried out using *Taq* DNA polymerase (1 U, Bioline) in *Taq* reaction buffer (as supplied), supplemented with 2 mM MgSO₄ and 0.2 mM dNTPs, using 25 thermal-cycling reactions as detailed above. Successful transformants generated a DNA fragment of approximately 500 bp, ascertained by 1.5 % agarose gel electrophoresis.

### Plasmid DNA preparation

Bacterial transformants that generated the correct size of PCR products are inoculated into 6 ml of sterile LB-kanamycin medium and incubated overnight at 37°C with 200 rpm shaking. pETBMC01 plasmid DNA is recovered from the bacterial cultures using a QIAprep Spin Mini-prep kit according to the manufacturer's instructions (Qiagen). The presence of the β2m fragment in these plasmids is further verified by automated DNA.

A schematic illustration of pETBMC01 is shown in Figure 3(b), specifically illustrating restriction sites.

### 1.2. Cloning COMP-BP into pETBMC01

### Synthesis of the COMP-BP cassette

The sequence of the oligomerisation domain of COMP is obtained from the Genbank database (accession #1705995) and a region encoding the coiled-coil domain (amino acids 21-85) is selected based on self-association experiments of COMP (Efimov et al., FEBS Letters 341:54-58 (1994)). A biotin acceptor sequence 'BP': SLNDIFEAQKIEWHE is incorporated at the C terminus and an additional 14 amino acid linker, PQPQPKPQPKPEPET is included to provide a physical separation between the COMP oligomerising domain and BP.

The whole region is synthesized using the overlapping complemantary oligonucleotides (BMC#3, BMC#4, BMC#5, BMC#6, BMC#7 and BMC#8) in a `PCR assembly' reaction. Oligonucleotide sequences are detailed in Appendix I and are synthesised and purified ('PAGE-pure') by Sigma-Genosys. The primers (4.8 pmoles) are phosphorylated with T4 kinase (10 U, Gibco) for 1 hour at 37°C, in forward reaction buffer (as supplied), supplemented with 1 mM ATP. The reaction is terminated by heating to 80°C for 15 min and then allowed to cool to room temperature, which enabled oligonucleotide annealling. The annealed oligonucleotides are ligated together using T4 DNA ligase as in Section 1.1, and ∼ 10 ng used in a PCR to 'fill in' the ends of the sequences and to amplify the synthetic gene. The PCR reaction is essentially as described in Section 1.1, except BMC#3 (15 µM) and BMC#8 (15 µM) are used as the forward and reverse primers in the following thermal cycling conditions: Step 1: 95 °C, 4 min; Step 2: 95°C, 1 min; Step 3: 70°C, 1 min; Step 4: 72°C, 2 min; Step 5: cycle to Step 2, 15x; Step 6: 72°C, 10 min. A PCR product of the correct size is gel purified as detailed previously (Section 1.1).

### Insertion of COMP-BP cassette into pETBMC01 to produce pETBMC02

200 ng of purified COMP-BP and 1 µg pETBMC01 vector are digested for 4 hrs at 37°C using *Hin*d III (10 U) and *Xho* I (10 U) restriction enzymes (NEB), as described in Section 1.1. The digestion products are purified, ligated, transformed and PCR screened as in Section 1.1. Plasmids positive from the screen are purified and sequenced as described in Section 1.1.

A schematic diagram of pETBMC02 is shown in Figure 3(c), illustrating restriction sites. The section encoding COMP-BP is out-of-frame in this construct.

### 1.3. Cloning the poly-glycine linker into pETBMC02

### Synthesis of the poly-glycine linker

The poly-glycine linker is synthesized by annealing overlapping oligonucleotides BMC#9 and BMC#10 (Sigma-Genosys, 'PAGE-pure', Appendix I). The poly-glycine linker comprises the following motif in single letter amino acid code: GS(GGGGS)₄GGK, when encoded in the correct reading frame. The first two residues are encoded by the *Bam*H I restriction site, whereas the last residue is encoded by the *Hin*d III restriction site. Since the nucleotide sequence of the polyGlycine linker only incorporates the 5' overhang of the cut *Bam*H I restriction site, and the 3' overhang of the cut *Hin*d III nucleotide recognition motifs, there is no need to digest the annealed product to produce the complementary single-stranded overhangs suitable for subsequent ligation. The oligonucleotides are phosphorylated and annealed as described in Section 1.2.

### Insertion of the poly-glycine linker into pETBMC02 to produce pETBMC03

pETBMC02 is digested with *Bam*H I (10U) and *Hin*d III (10 U) . Ligation of the annealed poly-glycine linker into pETBMC02 was as described previously (Section 1.1), assuming 96 fmoles of annealed oligonucleotide/µl. The transformation and PCR-screening reactions are as described in Section 1.1, but in addition, the presence of an inserted linker is verified by a restriction enzyme digestion of the PCR screen product to ascertain the presence or absence of a *Sal* I restriction site. Successful transformants are not susceptible to *Sal* I digestion, given the removal of the site from the plasmid vector backbone. Purification of pETBMC03 and automated sequencing is as described in Section 1.1.

A schematic diagram of pETBMC03 is shown in Figure 3(d), illustrating restriction sites. The *Bam*H I site encodes a serine residue immediately before the poly-glycine sequence. This serine residue is ultimately removed to give pETBMC04, as illustrated in 3(a).

### 1.4. Deletion of a serine residue immediately prior to the poly-glycine linker

### Site-directed mutagenesis of pETBMC03

Analysis of X-ray crystallography models of MHC class I molecules reveal that the C terminus of β2m closely abuts the β3 domain of the α chain. It is therefore desirable to achieve maximum flexibility at the start of the poly-glycine linker, by removal of the serine residue encoded by the *Bam*H I restriction motif, resulting in a linker comprising G(GGGGS)₄GGK. PCR primers BMC#11 and BMC#12 ('PAGE-pure', Appendix I) are obtained, which enabled removal of the TCC triplet that encodes serine within the *Bam* HI recognition motif, using a site-directed mutagenesis kit (`QuickChange', Stratagene) strictly in accordance with the manufacturer's instructions. Corrected versions of this construct are verified by automated sequencing (Section 1.1) and designated pETBMC04.

A schematic illustration of pETBMC04 is shown in Figure 3(a). Specifically this figure shows a schematic diagram of the completed β2m-COMP construct in pET24c (pETBMC04), illustrating the restriction sites.

### 2. Molecular cloning of the HLA-A*0201 α chain construct

### 2.1. Cloning A*0201 α chain into the pET-11d vector

The extracellular portion of HLA-A*0201 α chain (EMBL M84379) comprises of 276 amino acids (equivalent to Gly1-Pro276 of the mature protein) encoded by bases 73-900 of the messenger RNA sequence. This region of the A*0201 sequence is amplified from a normal human lymphocyte cDNA library by PCR, using the primers A2S#1 and A2S#2 which incorporated *NcoI* and *BamH*I restriction sites respectively. The procedure for cloning the A*0201 insert into *Nco I*/*BamH* I-digested pET-11d vector (Novagen) is essentially as described for β2m in Section 1.1.

The resulting plasmid is named pETA2sol, and a schematic diagram is shown in Figure 3(e), illustrating restriction sites.

### 3. Expression of β2m-COMP and HLA-A*0201 α chain proteins

An identical procedure is carried out to produce either β2m-COMP or A*0201 α chain proteins. Plasmid DNA is transformed into an *E. coli* expression host strain in preparation for a large scale bacterial prep. Protein is produced as insoluble inclusion bodies within the bacterial cells, and is recovered by sonication. Purified inclusion bodies are solubilised in denaturing buffer and stored at -80°C until required. Unless specified, chemical reagents are obtained from Sigma.

### 3.1. Large scale production of recombinant proteins

### Transformation of pETBMC04 and pETA2sol into E. coli host strain BL21(DE3)pLysS

Purified plasmid DNA is transformed into the BL21(DE3)pLysS *E. coli* strain, which carries a chromosomal copy of the T7 RNA polymerase required to drive protein expression from pET-based constructs. Transformations into BL21(DE3)pLysS competent cells (Stratagene) are carried out with appropriate controls, according to the manufacturer's instructions, using 0.5µg purified plasmid DNA for each reaction. Successful transformants are selected on LB-agar plates containing 34 µg/ml chloramphenicol, in addition to kanamycin 30µg/ml (pETBMC04) or 100µg/ml ampicillin (pETA2sol).

### Growth of E. coli cultures

A single bacterial transformant colony is innoculated into 60ml sterile LB medium, containing appropriate antibiotics for selection, and left to stand overnight in a warm room (∼24°C). The resulting overnight culture is added to 6 litres of LB and grown at 37°C with shaking (∼240 rpm), up to mid-log phase (OD₆₀₀ = 0.3-0.4). Protein expression is induced at this stage by addition of 1.0 ml of 1M IPTG to each flask. The cultures are left for a further 4 h at 37°C with shaking, after which the cells are harvested by centrifugation and the supernatant discarded.

### Sonication and recovery of proteins

The bacterial cell pellet is resuspended in ice-cold balanced salt solution and sonicated (*XL* series sonicator; Misonix Inc., USA) in a small glass beaker on ice in order to lyse the cells and release the protein inclusion bodies. Once the cells are completely lysed the inclusion bodies are spun down in 50 ml polycarbonate Oak Ridge centrifuge tubes in a Beckman high-speed centrifuge (*J*2 series) at 15,000 rpm for 10 min. The inclusion bodies are then washed three times in chilled Triton wash buffer (0.5% Triton X-100, 50 mM Tris, pH 8.0, 100 mM sodium chloride, 0.1 % sodium azide and freshly added 2 mM dithiothreitol [DTT]), using a hand-held glass homogeniser to resuspend the pellet after each wash. This is followed by a final wash in detergent-free wash buffer.

The resultant purified protein preparation is solubilised in 20-50 ml of 8 M urea buffer, containing 50 mM MES, pH 6.5, 0.1 mM EDTA and 1 mM DTT, and left on an end-over-end rotator overnight at 4°C. Insoluble particles are removed by centrifugation and the protein yield is determined using Bradford's protein assay reagent (Bio-Rad Laboratories) and by comparison with known standards. The quality of expression and purification is also verified by vertical electrophoresis of samples on a 15% sodium dodecyl sulphate-polyacrylamide gel (SDS-PAGE). Typical protein yields are 25-75 mg per litre of LB culture. The β2m-COMP and A*0201 α chain proteins are approximately 27 (kD) and 35 kD in size, respectively. Urea-solubilised protein is dispensed in 10mg aliquots and stored at -80°C for future use.

### 4. Formation of pentameric β2m-COMP complexes

### 4.1. Assembly of β2m-COMP into pentamers

Assembly of β2m-COMP from the urea-solubilised inclusion bodies is performed by diluting the protein into 20 mM CAPS buffer, pH 11.0, containing 0.2 M sodium chloride and 1 mM EDTA, to give a final protein concentration of 1.5 mg/ml. The protein is oxidised at room temperature by addition of oxidised and reduced glutathione to final concentrations of 20 mM and 2 mM, respectively. Following an overnight incubation, disulphide bond formation is analysed by non-reducing SDS-PAGE on 10 % bis-tricine gels (Invitrogen).

The protein mixture is subsequently buffer exchanged into 20 mM Tris, pH 8.0, 50 mM sodium chloride ('S200 buffer'), using Centricon Plus-20 ultra-centrifugal concentrators (10 kD molecular weight cut-off, Milllipore) according to the manufacturer's instructions, and concentrated to a final volume of 4.5 ml, in preparation for enzymatic biotinylation with BirA (Affinity, Denver, Colorado). 0.5 ml of 10x BirA reaction buffer (as supplied) is added, and recombinant BirA enzyme at 10 µM final concentration, supplemented with 10 mM ATP, pH 7.0. A selection of protease inhibitors is also used to preserve the proteins: 0.2 mM PMSF, 2 µg/ml pepstatin and 2 µg/ml leupeptin. The reaction is left for 4 hours at room temperature.

### 4.2. Purification of biotinylated β2m-COMP pentamers

Biotinylated β32m-COMP is purified by size exclusion chromatography (SEC) on a Superdex 200 HR 26/60 column (Amersham Biosciences), running S200 buffer. The column is previously calibrated and used according to the manufacturer's instructions and fractions containing pentamers in the region of 135 kD are collected. Protein recovery is determined using the Bradford Assay, and biotinylation efficiency is determined by use of HABA reagent (Pierce), according to manufacturer's instructions.

### 5. Formation of refolded HLA-A*0201/GLCTLVAML complexes

### 5.1. Refolding HLA-A *0201 α chain with biotinylated β2m-COMP and peptide

500 ml of refolding buffer is prepared as follows: 100 mM Tris, pH 8.0, 400 mM L-arginine hydrochloride, 2 mM EDTA, 5 mM reduced glutathione and 0.5 mM oxidised glutathione, dissolved in deionised water and left stirring at 4°C. 15 mg of lyophilised synthetic peptide GLCTLVAML is dissolved in 0.5 ml dimethylsulfoxide and added to the refolding buffer whilst stirring. 50 mg of biotinylated pentameric β2m-COMP and 30 mg of A*0201 α chain is added sequentially, injected through a 23gauge hypodermic needle directly into the vigorously-stirred buffer, to ensure adequate dispersion. The refolding mixture is then left stirring gently for 16 hours at 4°C.

### 5.2. Purification of refolded pentameric HLA-A*0201/GLCTLVAML complexes

The protein refolding mixture is subsequently concentrated from 500 ml to 20 ml using a MiniKros hollow fibre ultrafiltration cartridge (Spectrum Labs, Rancho Dominguez, California) with a 30 kD molecular weight cutoff. Further concentration of the complex from 20 ml to 5 ml is carried out in Centricon Plus-20 centrifugal concentrators (30 kD molecular weight cut-off) according to the manufacturers instructions, followed by buffer exchange into S200 buffer using disposable PD10 desalting columns (Amersham Biosciences), according to the manufacturer's instructions. Final volume is 7.5 ml. The concentrated protein refold mixture is first purified by SEC on a Superdex 200 HR 26/60 gel filtration chromatography column, as in Section 4.2. Fractions containing protein complexes in the region of 310 kD is collected.

Fractions collected from SEC are pooled and subjected to further purification by anion exchange chromatography on a MonoQ HR 5/5 column (Amersham Biosciences), running a salt gradient from 0 - 0.5 M sodium chloride in 20 mM Tris over 15 column volumes. The dominant peak is collected. Protein recovery is determined using the Bradford assay.

### 5.3. Fluorescent labelling of purified complexes

Since each streptavidin molecule is able to bind up to 4 biotin entities, final labelling with phycoerythrin (PE)-conjugated streptavidin is carried out in a molar ratio of 1:0.8, streptavidin to biotinylated pentamer complex respectively, taking into account the initial biotinylation efficiency measurement made for β2m-COMP in Section 4.2. The total required amount of pentamer complex is subdivided (e.g. into 5 equal amounts) and titrated successively into streptavidin-PE. The concentration of A*0201 pentamer-streptavidin complex is adjusted to 1 mg/ml with phosphate buffered saline (PBS), supplemented with 0.01 % azide and 1 % BSA. This resultant fluorescent reagent is stored at 4°C.

### 6. Staining of antigen-specific T cells

Peripheral blood lymphocytes (PBL) are obtained by drawing a blood sample from a healthy, A*0201-positive, EBV carrier who had previously been shown to elicit strong cytotoxic T cell responses to the GLCTLVAML antigen. The PBL are isolated by density gradient centrifugation on Ficoll (Amersham Biosciences), washed in PBS solution and counted. 1-2 x 10⁶ lymphocytes are allocated for each staining condition and the cells are washed once in 1 ml wash buffer (0.1 % sodium azide, 0.1 % BSA in PBS) and then resuspended in 50 □1 of wash buffer. 1 □g of PE-labelled A*0201/GLCTLVAML pentamer used to stain a single aliquot of cells, in combination with an anti-CD8 antibody (Dako), to identify the cytotoxic T cell population. The suspension is incubated on ice for at least 30 minutes in the dark, after which the cells are washed twice in wash buffer and stored in fix solution in the dark (1 % foetal calf serum, 2.5 % formaldehyde in PBS). Thereafter samples are analysed on a Becton-Dickinson *FACScan*^{™} flow cytometer, using CellQuest software, according to standard methods well known in the art.

**Appendix I: Table of primers used for synthesis of DNA constructs**

| **Primer name** | **Sequence ID No.** | **Direction** | **Sequence (5'-3')** |
|---|---|---|---|
| BMC #1 | 1 | Forward | |
| | | | |
| BMC #2 | 2 | Reverse | |
| | | | |
| BMC #3 | 3 | Forward | |
| | | | |
| BMC #4 | 4 | Reverse | |
| | | | |
| BMC #5 | 5 | Forward | |
| | | | |
| BMC #6 | 6 | Reverse | |
| | | | |
| BMC #7 | 7 | Forward | |
| | | | |
| BMC #8 | 8 | Reverse | |
| | | | |
| BMC #9 | 9 | Forward | |
| | | | |
| BMC #10 | 10 | Reverse | |
| | | | |
| BMC #11 | 11 | Forward | GAG ACA TGG GAG GTG GTG GTG G |
| | | | |
| BMC #12 | 12 | Reverse | CCA CCA CCA CCT CCC ATG TCT C |
| | | | |
| A2S #1 | 13 | Forward | |
| | | | |
| A2S #2 | 14 | Reverse | |
| | | | |
| T7 promoter | 15 | Forward | TAA TAC GAC TCA CTA TAG GG |
| | | | |
| T7 terminator | 16 | Reverse | GCT AGT TAT TGC TCA GCG G |
| | | | |

## Claims

1. Oligomeric MHC complex comprising at least two chimeric proteins, said chimeric proteins comprising a first section derived from an MHC peptide chain or a functional part thereof and a second section comprising a pentamerisation domain derived from the pentamerisation domain of a cartilage oligomeric matrix protein (COMP), wherein formation of the oligomeric MHC complex occurs by oligomerisation at the pentamerisation domain of the chimeric proteins, and wherein at least two of the first sections are derived from the same MHC peptide chain.

2. Oligomeric MHC complex of claim 1 wherein the first section of the chimeric proteins is derived from the extra-cellular part of the MHC class I or II α chain.

3. Oligomeric MHC complex of any one of the preceding claims wherein the first section of the chimeric proteins is derived from the extra-cellular part of the MHC class I or II β chain.

4. Oligomeric MHC complex of any one of the preceding claims wherein the pentamerisation domain of COMP comprised in the second section in at least one of the chimeric proteins comprises and preferably consists of amino acids 1 to 128 of COMP, preferably 21 to 85 of human COMP.

5. Oligomeric MHC complex of any one of the preceding claims wherein at least one of the chimeric proteins further comprises a first linker between the MHC peptide chain and the pentamerisation domain.

6. Oligomeric MHC complex of any one of the preceding claims wherein at least one of the chimeric proteins further comprises one or more domains selected from the group consisting of a second linker, a tagging domain and a purification domain.

7. Oligomeric MHC complex according to any one of the preceding claims further comprising the complementary MHC peptide chains to at least two of the chimeric proteins to form functional MHC binding complexes.

8. Oligomeric MHC complex according to claim 7 further comprising peptide bound to the MHC portions of the complex in the groove formed by the MHC α1 and α2 domains for class I complexes or the MHC α1 and β1 domains for class II complexes.

9. Oligomeric MHC complex according to claim 8 wherein the peptide is substantially homogeneous.

10. Oligomeric MHC complex according to any one of the preceding claims comprising a label.

11. Oligomeric MHC complex according to claim 10 wherein the label is selected from the group consisting of a light detectable label, a radioactive label, an enzyme, an epitope, a lectin, or biotin.

12. Chimeric protein comprising a first section which is an MHC peptide chain or a functional part thereof and a second section comprising a pentamerisation domain derived from the pentamerisation domain of cartilage oligomeric matrix protein (COMP).

13. Chimeric protein according to claim 12 wherein the pentamerisation domain comprises and preferably consists of the amino acids 1 to 128 of COMP, preferably 21 to 85, of human COMP.

14. A recombinant expression cassette comprising a promoter sequence operably linked to a nucleotide sequence coding for a chimeric protein as defined in any one of claims 12 or 13.

15. Vector comprising the recombinant expression cassette of claim 14.

16. Pharmaceutical or diagnostic composition, comprising an oligomeric MHC complex according to any one of claims 1 to 11, optionally in combination with a pharmaceutically acceptable carrier.

17. Method of labeling and or detecting mammalian T cells according to the specificity of their antigen receptor, the method comprising
(i) combining an oligomeric MHC complex according to any one of claims 1 to 11 and a suspension or biological sample comprising T cells, and
(ii) detecting the presence of specific binding of said complex and the T cells.

18. Method of separating mammalian T cells according to the specificity of their antigen receptor, the method comprising
(i) combining an oligomeric MHC complex according to any one of claims 1 to 11 and a suspension or biological sample comprising T cells, and
(ii) separating T cells bound to said complex from unbound cells.

## Patentansprüche

1. Oligomerer MHC-Komplex, umfassend mindestens zwei chimäre Proteine, wobei die chimären Proteine einen ersten Abschnitt umfassen, der von einer MHC-Peptidkette oder einem funktionalen Teil davon abgeleitet ist, und einen zweiten Abschnitt umfassen, der eine Pentamerisationsdomäne umfasst, die von der Pentamerisationsdomäne des Cartilage Oligomeric Matrix-Proteins (COMP) abgeleitet ist, wobei der oligomere MHC-Komplex durch die Oligomerisation an der Pentamerisationsdomäne der chimären Proteine gebildet wird, und wobei mindestens zwei der ersten Abschnitte von der gleichen MHC-Peptidkette abgeleitet sind.

2. Oligomerer MHC-Komplex nach Anspruch 1, wobei der erste Abschnitt der chimären Proteine von einem extrazellulären Teil der MHC-Klasse I- oder II-α-Kette abgeleitet ist.

3. Oligomerer MHC-Komplex nach einem der vorstehenden Ansprüche, wobei der erste Abschnitt der chimären Proteine von einem extrazellulären Teil der MHC-Klasse I- oder II-β-Kette abgeleitet ist.

4. Oligomerer MHC-Komplex nach einem der vorstehenden Ansprüche, wobei die Pentamerisationsdomäne des COMP, die in dem zweiten Abschnitt in mindestens einem der chimären Proteine umfasst ist, Aminosäuren 1 bis 128 des COMPs, vorzugsweise 21 bis 85 des menschlichen COMPs, umfasst und vorzugsweise aus diesen besteht.

5. Oligomerer MHC-Komplex nach einem der vorstehenden Ansprüche, wobei mindestens eines der chimären Proteine weiterhin einen ersten Linker zwischen der MHC-Peptidkette und der Pentamerisationsdomäne umfasst.

6. Oligomerer MHC-Komplex nach einem der vorstehenden Ansprüche, wobei mindestens eines der chimären Proteine weiterhin eine oder mehrere Domänen umfasst, ausgewählt aus der Gruppe bestehend aus einem zweiten Linker, einer Markierungsdomäne und einer Reinigungsdomäne.

7. Oligomerer MHC-Komplex nach einem der vorstehenden Ansprüche, weiterhin umfassend die komplementären MHC-Peptidketten zu mindestens zweien der chimären Proteine, um funktionale MHC-Bindungskomplexe zu bilden.

8. Oligomerer MHC-Komplex nach Anspruch 7, weiterhin umfassend ein Peptid, das an die MHC-Teile des Komplexes in der Furche gebunden ist, der durch die MHC-α1- oder MHC-α2-Domänen für Klasse I-Komplexe oder die MHC-α1- und MHC-β1-Domänen für Klasse II-Komplexe gebildet wird.

9. Oligomerer MHC-Komplex nach Anspruch 8, wobei das Peptid im Wesentlichen homogen ist.

10. Oligomerer MHC-Komplex nach einem der vorstehenden Ansprüche, umfassend eine Markierung.

11. Oligomerer MHC-Komplex nach Anspruch 10, wobei die Markierung ausgewählt ist aus der Gruppe bestehend aus einer mit Licht nachweisbaren Markierung, einer radioaktiven Markierung, einem Enzym, einem Epitop, einem Lectin oder Biotin.

12. Chimäres Protein, umfassend einen ersten Abschnitt, der eine MHC-Peptidkette oder ein funktionaler Teil davon ist, und einen zweiten Abschnitt, umfassend eine Pentamerisationsdomäne, die von der Pentamerisationsdomäne des Cartilage Oligomeric Matrix-Proteins (COMP) abgeleitet ist.

13. Chimäres Protein nach Anspruch 12, wobei die Pentamerisationsdomäne die Aminosäuren 1 bis 128 des COMPs, vorzugsweise 21 bis 85 des menschlichen COMPs, umfasst und vorzugsweise aus diesen besteht.

14. Rekombinante Expressionskassette, umfassend eine Promotorsequenz, die mit einer Nucleotidsequenz funktionell verknüpft ist, die ein chimäres Protein wie in einem der Ansprüche 12 oder 13 definiert codiert.

15. Vektor, umfassend die rekombinante Expressionskassette nach Anspruch 14.

16. Pharmazeutische oder diagnostische Zusammensetzung, umfassend einen oligomeren MHC-Komplex nach einem der Ansprüche 1 bis 11, gegebenenfalls in Kombination mit einem pharmazeutisch verträglichen Träger.

17. Verfahren zur Markierung und/oder zum Nachweis von Säuger-T-Zellen nach der Spezifität ihres Antigenrezeptors, wobei das Verfahren umfasst
(i) das Kombinieren eines oligomeren MHC-Komplexes nach einem der Ansprüche 1 bis 11 und einer T-Zellen umfassenden Suspension oder biologischen Probe, und
(ii) das Nachweisen des Vorhandenseins einer spezifischen Bindung des Komplexes und der T-Zellen.

18. Verfahren zur Trennung von Säuger-T-Zellen nach der Spezifität ihres Antigenrezeptors, wobei das Verfahren umfasst
(i) das Kombinieren eines oligomeren MHC-Komplexes nach einem der Ansprüche 1 bis 11 und einer Suspension oder biologischen Probe umfassend T-Zellen, und
(ii) das Trennen der an den Komplex gebundenen T-Zellen von ungebundenen Zellen.

## Revendications

1. Complexe CMH oligomère comprenant aux moins deux protéines chimériques, lesdites protéines chimériques comprenant une première section dérivée d'une chaîne peptidique du CMH ou d'une partie fonctionnelle de celle-ci et une seconde section comprenant un domaine de pentamérisation dérivé du domaine de pentamérisation de la COMP (cartilage oligomeric matrix protein), où la formation du complexe CMH oligomère se produit par oligomérisation au niveau du domaine de pentamérisation des protéines chimériques, et où au moins deux des premières sections sont dérivées de la même chaîne peptidique du CMH.

2. Complexe CMH oligomère selon la revendication 1, dans lequel la première section des protéines chimériques est dérivée de la partie extracellulaire de la chaîne α du CMH de classe I ou II.

3. Complexe CMH oligomère selon l'une quelconque des revendications précédentes, dans lequel la première section des protéines chimériques est dérivée de la partie extracellulaire de la chaîne β du CMH de classe I ou II.

4. Complexe CMH oligomère selon l'une quelconque des revendications précédentes, dans lequel le domaine de pentamérisation de la COMP compris dans la seconde section dans au moins l'une des protéines chimériques comprend et est constitué de préférence des acides aminés 1 à 128 de la COMP, de préférence 21 à 85 de la COMP humaine.

5. Complexe CMH oligomère selon l'une quelconque des revendications précédentes, dans lequel au moins l'une des protéines chimériques comprend en outre un premier lieur entre la chaîne peptidique du CMH et le domaine de pentamérisation.

6. Complexe CMH oligomère selon l'une quelconque des revendications précédentes, dans lequel au moins l'une des protéines chimériques comprend en outre un ou plusieurs domaines choisis dans le groupe constitué d'un second lieur, d'un domaine de marquage et d'un domaine de purification.

7. Complexe CMH oligomère selon l'une quelconque des revendications précédentes comprenant en outre les chaînes peptidiques du CMH complémentaires d'au moins deux des protéines chimériques pour former des complexes fonctionnels de liaison du CMH.

8. Complexe CMH oligomère selon la revendication 7 comprenant en outre un peptide lié aux parties du CMH du complexe dans la poche formée par les domaines α1 et α2 du CMH pour les complexes de classe I ou les domaines α1 et β2 du CMH pour les complexes de classe II.

9. Complexe CMH oligomère selon la revendication 8, dans lequel le peptide est pratiquement homogène.

10. Complexe CMH oligomère selon l'une quelconque des revendications précédentes comprenant un marqueur.

11. Complexe CMH oligomère selon la revendication 10, dans lequel le marqueur est choisi dans le groupe constitué d'un marqueur détectable par la lumière, d'un marqueur radioactif, d'une enzyme, d'un épitope, d'une lectine ou de la biotine.

12. Protéine chimérique comprenant une première section qui est une chaîne peptidique du CMH ou une partie fonctionnelle de celle-ci et une seconde section comprenant un domaine de pentamérisation dérivé du domaine de pentamérisation de la COMP (cartilage oligomeric matrix protein).

13. Protéine chimérique selon la revendication 12, dans laquelle le domaine de pentamérisation comprend et est constitué de préférence des acides aminés 1 à 128 de la COMP, de préférence 21 à 85 de la COMP humaine.

14. Cassette d'expression recombinante comprenant une séquence promoteur liée de manière fonctionnelle à une séquence nucléotidique codant pour une protéine chimérique telle que définie dans l'une quelconque des revendications 12 ou 13.

15. Vecteur comprenant la cassette d'expression recombinante selon la revendication 14.

16. Composition pharmaceutique ou diagnostique, comprenant un complexe CMH oligomère selon l'une quelconque des revendications 1 à 11, éventuellement en combinaison avec un support pharmaceutiquement acceptable.

17. Procédé de marquage et/ou de détection de cellules T mammaliennes selon la spécificité de leur récepteur d'antigène, le procédé comprenant
(i) la combinaison d'un complexe CMH oligomère selon l'une quelconque des revendications 1 à 11 et d'une suspension ou d'un échantillon biologique comprenant des cellules T, et
(ii) la détection de la présence d'une liaison spécifique dudit complexe et des cellules T.

18. Procédé de séparation de cellules T mammaliennes selon la spécificité de leur récepteur d'antigène, le procédé comprenant
(i) la combinaison d'un complexe CMH oligomère selon l'une quelconque des revendications 1 à 11 et d'une suspension ou d'un échantillon biologique comprenant des cellules T, et
(ii) la séparation des cellules T liées au dit complexe des cellules non liées.


### SEQUENCE LISTING

<110> ProImmune Limited
   <120> Chimeric Protein and Oligomer Thereof
   <130> PI03
   <160> 16
   <170> PatentIn version 3.1
<210> 1
   <211> 38
   <212> DNA
   <213> Artificial
   <220>
   <223> Oligonucleotide (Forward)
   <400> 1
   gcatcaccat atgatccagc gtactccaaa gattcagg 38
<210> 2
   <211> 36
   <212> DNA
   <213> Artificial
   <220>
   < 223> Oligonucleotide (Reverse)
   <400> 2
   ctacaaggat cccatgtctc gatcccactt aactat 36
<210> 3
   <211> 80
   <212> DNA
   <213> Artificial
<220>
   <223> Oligonucleotide (Forward)
   <400> 3
   taaagcttca gggccagagc ccgttgggct cagacctggg cccgcagatg cttcgggaac 60
   tgcaggaaac caacgcggcg 80
<210> 4
   <211 > 81
   <212> DNA
   <213> Artificial
   <220>
   <223> Oligonucleotide (Reverse)
   <400> 4
   gaacgtgatc tccctgacct gctgccgcag cagctcccgc acgtcctgca gcgccgcgtt 60
   ggtttcctgc agttcccgaa g 81
<210> 5
   <211 > 81
   <212> DNA
   <213> Artificial
   <220>
   <223> Oligonucleotide (Forward)
   <400> 5
   ctgcaggacg tccgggagct gctgcggcag caggtcaggg agatcacgtt cctgaaaaac 60
   acggtgatgg agtgtgacgc g 81
<210> 6
   <211> 80
   <212> DNA
   <213> Artificial
   <220>
   <223> Oligonucleotide (Reverse)
   <400> 6
   tacggccgca cgctgggtag gccggtgcgt actgactgct gcatcccgca cgcgtcacac 60
   tccatcaccg tgtttttcag 80
<210> 7
   <211> 108
   <212> DNA
   <213> Artificial
   <220>
   <223> Oligonucleotide (Forward)
   <400> 7
   tgcgggatgc agcagtcagt acgcaccggc ctacccagcg tacggccgcc gcagccgcag 60
   ccgaaaccgc agccgaaacc ggaaccggaa actagtttga acgacatc 108
<210> 8
   <211> 96
   <212> DNA
   <213> Artificial
   <220>
   <223> Oligonucleotide (Reverse)
   <400> 8
   tactcgagtt cgtgccattc gattttctga gcctcgaaga tgtcgttcaa actagtttcc 60
   ggttccggtt tcggctgcgg tttcggctgc ggctgc 96
<210> 9
   <211> 72
   <212> DNA
   <213> Artificial
   <220>
   <223> Oligonucleotide (Forward)
   <400> 9
   gatccggtgg tggtggttct ggtggtggtg gttctggtgg tggtggttct ggtggtggtg 60
   gttctggtgg ta 72
<210> 10
   <211 > 72
   <212> DNA
   <213> Artificial
   <220>
   <223> Oligonucleotide (Reverse)
   <400> 10
   agcttaccac cagaaccacc accaccagaa ccaccaccac cagaaccacc accaccagaa 60
   ccaccaccac cg 72
<210> 11
   <211> 22
   <212> DNA
   <213> Artificial
   <220>
   <223> Oligonucleotide (Forward)
   <400> 11
   gagacatggg aggtggtggt gg 22
<210> 12
   <211 > 22
   <212> DNA
   <213> Artificial
   <220>
   < 223> Oligonucleotide (Reverse)
   <400> 12
   ccaccaccac ctcccatgtc tc 22
<210> 13
   <211> 35
   <212> DNA
   <213> Artificial
   <220>
   < 223 > Oligonucleotide (Forward)
   <400> 13
   gcatcaccat gggttctcac tctatgaggt atttc 35
<210> 14
   <211> 37
   <212> DNA
   <213> Artificial
   <220>
   <223> Oligonucleotide (Reverse)
   <400> 14
   gcatacggat ccttacggct cccatctcag ggtgagg 37
<210> 15
   <211> 20
   <212> DNA
   <213> Artificial
   <220>
   <223> Oligonucleotide (Forward)
   <400> 15
   taatacgact cactataggg 20
<210> 16
   <211> 19
   <212> DNA
   <213> Artificial
   <220>
   <223> Oligonucleotide (Reverse)
   <400> 16
   gctagttatt gctcagcgg 19